# EUROPÄISCHE PATENTANMELDUNG

(11) **EP 1 649 888 A2**
(43) Veröffentlichungstag der Anmeldung: **26.04.2006**
(21) Anmeldenummer: 05022628.1
(22) Anmeldetag: 18.10.2005
(51) Int. Cl.: A61M 16/04, A61M 39/02, A61M 1/00, A61F 5/445

(54) **Gewebekonnektor**

(30) Priorität: 25.10.2004 DE 102004052408
(71) Anmelder: Adeva Medical Gesellschaft für Entwicklung und Vertrieb von Medizinischen Implantat-Artikeln mbH, 23556 Lübeck (DE)
(72) Erfinder: Grundei, Hans, Dr.-Ing., 23558 Lübeck (DE)
(74) Vertreter: Fuchs Mehler Weiss & Fritzsche

(57) **Zusammenfassung**

Es wird ein Gewebekonnektor (1) zur Ausbildung eines infektfreien Hautdurchtrittes beschrieben, der zum Anschluss extrakorporaler Hilfsmittel dient.

Der Konnektor (1) weist einen flächigen Träger (2) aus einem implantierbaren zweidimensionalen Netz oder einem dreidimensionalen Mesh auf. Er fasst einen flexiblen Kernring (3) ein, der als Ankoppelungselement für die Hilfsmittel dient.

## Beschreibung

Die Erfindung betrifft einen Gewebekonnektor zur Ausbildung eines infektfreien Hautdurchtrittes.

Grundsätzlich ist es problematisch, extrakorporale Hilfsmittel zu applizieren, die mit dem Körperinneren in Verbindung stehen. Namentlich ist die Verkeimungsgefahr des Durchtritts durch die Haut des Patienten das größte Problem.

### Als Beispiele seien die folgenden Anwendungsfälle angegeben:

Bei der Rehabilitation laryngektomierter Personen wird ein künstliches Stoma, durch welches der Patient atmen kann, operativ geschaffen. Mit diesem Stoma ist ein Hilfsmittel wie ein Tracheostoma-Ventil, beispielsweise gemäß der EP 1 025 874 B1 oder gemäß der EP 0 617 630 B1, koppelbar. Hierbei kommt es darauf an, die Hilfsmittel ohne die bei konventionellen Tracheostomata häufig auftretenden Probleme wie Leckagen und Gewebereizungen zu adaptieren.

Ein weiterer Anwendungsfall für einen Gewebekonnektor ist beispielsweise ein Anus praeter, also ein künstlicher Darmausgang. Es handelt sich hierbei um einen operativ angelegten doppelläufigen oder endständigen Darmausgang im Bereich des Abdomens zur Stuhlentleerung in einen Auffangbeutel. Hier bedarf es einer Ankoppelung eines Ableitungsschlauches an die Darmfistel. Ein noch weiteres Anwendungsgebiet ist die Enterostomie, also eine endoskopische oder operativ angelegte perkutan ausgeleitete Fistel zum Magen-Darmtrakt, z.B. als Emährungsfistel in Form einer perkutanen endoskopischen Gastronomie oder einer endoskopisch kontrollierten perkutanen Jejunostomie.

Ein noch weiteres Anwendungsgebiet ist der Hautdurchtritt einer Endo-Exo-Endoprothese, wie sie beispielsweise bekannt ist aus der EP 1 087 733 B1. Hier ist in einem Röhrenknochenstumpf ein Adapter mit seinem Stielteil implantiert, wobei dieses an seinem anderen Ende mit einer Koppelungseinrichtung für ein exoprothetisches Standardteil versehen ist. Das distale Ende tritt durch den Gliedmaßenstumpf hindurch, es kommt also zu einem Hautdurchtritt. Als extrakorporales Hilfsmittel kommt hier ein exoprothetisches Standardteil zur Anwendung. Die Durchtrittsstelle durch die Haut ist in diesem Falle besonders verkeimungsgefährdet. In der besagten Druckschrift ist als Keimsperre ein auf dem distalen Ende des Adapters sitzender Ring vorgesehen.

Vor diesem Hintergrund ist es die Aufgabe der vorliegenden Erfindung, einen Gewebekonnektor zur Ausbildung eines infektfreien Hautdurchtrittes auf allen genannten und weiteren Gebieten der Medizin vorzuschlagen, welcher eine hohe Sicherheit gegenüber einer Verkeimung und eine hohe Langzeitstabilität aufweist.

Gelöst wird diese Aufgabe durch einen Gewebekonnektor gemäß den nebengeordneten Ansprüchen 1 und 2.

Gemäß dem ersten Vorschlag ist vorgesehen, dass der Gewebekonnektor einen flächigen Träger aus einem implatierbaren zweidimensionalen Netz aufweist, der einen Kernring einfasst, der als Ankoppelungselement für die Hilfsmittel dient.

Das zweidimensionale Netz, welches subkutan zu implantieren ist, bietet eine äußerst flexible Verbindung mit der Haut und dem umgebenden Gewebe, nachdem dieses durch das Netz hindurchgranuliert ist.

Die eigentliche Abdichtung gegen eine Verkeimung bildet der flexible Kemring. In diesen wird das betreffende Hilfsmittel mit seinem Anschlussstück gesetzt, wobei die Dimensionen so gewählt sind, dass der Kernring das Anschlussstück des Hilfsmittels fest umgreift. Das Anschlussstück ist bei einem Tracheostoma-Ventil ein Tracheostoma-Kanülenansatz, beim Anus praeter ein entsprechender Anschlussschlauch eines Auffangbeutels und im Falle einer Endo-Exo-Prothese das distale Ende des beschriebenen Adapters.

Gemäß einem zweiten Vorschlag ist der Gewebekonnektor gemäß dem ersten Vorschlag so modifiziert, dass der Träger aus einem dreidimensionalen Mesh besteht. Unter einem Mesh wird in diesem Zusammenhang ein Aufbau verstanden, welcher jenem einer (metallischen) Wolle entspricht. Hier granuliert die Haut und das Gewebe nicht nur in der Fläche, sondern auch in der Tiefe ein, so dass der Sitz des Trägers gegenüber dem Netz des ersten Vorschlags sehr viel strammer ist, dafür jedoch nicht mehr so flexibel. Die Wahl des Gewebekonnektors nach dem ersten oder nach dem zweiten Vorschlag hängt unter anderem ab vom Ort der Implantation und von den Patientenwünschen.

Gemäß einer vorteilhaften Weiterbildung ist vorgesehen, dass der Kernring aus Silikon besteht. Silikon ist allgemein als körperverträglicher Kunststoff bekannt.

Gemäß einer vorteilhaften Weiterbildung besteht der Träger aus Polypropylen. Dies kann besonders bevorzugt silikonisiert oder titanisiert sein. Das Polypropylen kann auch mit Hydroxylapatit beschichtet sein.

Alternativ hierzu kann der Gewebekonnektor einen Träger aufweisen, der aus körperverträglichem Metall besteht. Hier kommt bevorzugt Titan zum Einsatz.

Um den Träger möglichst flexibel zu halten, sind in besonders bevorzugter Ausführungsform in die Umfangskante des Trägers radiale Schlitze eingelassen. Dies gestattet die Anpassung des Trägers an die örtlichen Gegebenheiten der Implantationsstelle.

Nachfolgend wird die Erfindung anhand eines Ausführungsbeispiels näher erläutert. Hierbei zeigt:
- Figur 1: eine Aufsicht auf den Gewebekonnektor,
- Figur 2: eine Schnittansicht entlang der Linie II-II in Figur 1, und
- Figur 3: die vergrößerte Ansicht der Einzelheit Z in Figur 2.

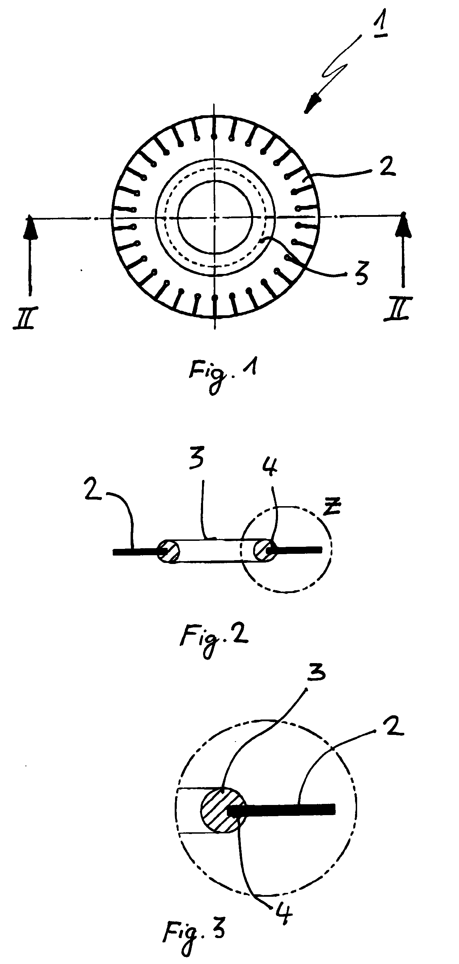

Wie aus Figur 1 ersichtlich besteht der Gewebekonnektor 1 zunächst aus einem flachen Träger 2. Im Inneren des Gewebekonnektors 1 befindet sich der flexible Kernring 3. Dieser wird von dem Träger 2 eingefasst. Zu diesem Zweck weist der Kernring eine eingelassene umlaufende Nut 4 auf, in welche der Träger 2 greift, wie aus Figur 2 und insbesondere Figur 3 deutlich wird. Der Kernring 3 hat einen im wesentlichen kreisförmigen Querschnitt. Er besteht aus einem flexiblen Material, vorzugsweise Silikon. Wenn ein Anschlussstück eines Hilfsmittels (nicht dargestellt) in den Ring 3 gesetzt wird, sorgen Rückstellkräfte des Kemringes 3 für eine feste Anlage an das Anschlussstück des Hilfsmittels und so für eine keimfreie Verbindung.

## Patentansprüche

1. Gewebekonnektor (1) zur Ausbildung eines infektfreien Hautdurchtrittes, der zum Anschluss extrakorporaler Hilfsmittel dient, mit einem flächigen Träger (2) aus einem implantierbaren zweidimensionalen Netz, der einen flexiblen Kernring (3) einfasst, der als Ankoppelungselement für die Hilfsmittel dient.

2. Gewebekonnektor (1) zur Ausbildung eines infektfreien Hautdurchtrittes, der zum Anschluss extrakorporaler Hilfsmittel dient, mit einem flächigen Träger (2) aus einem implantierbaren dreidimensionalen Mesh, der einen flexiblen Kernring (3) einfasst, der als Ankoppelungselement für die Hilfsmittel dient.

3. Gewebekonnektor nach Anspruch 1 oder 2, bei dem der Kernring (3) aus Silikon besteht.

4. Gewebekonnektor nach einem der Ansprüche 1 bis 3, bei dem der Träger (2) aus Polypropylen besteht.

5. Gewebekonnektor nach Anspruch 4, bei dem das Polypropylen silikonisiert ist.

6. Gewebekonnektor nach Anspruch 4, bei dem das Polypropylen titanisiert ist.

7. Gewebekonnektor nach Anspruch 4, bei dem der Träger mit Hydroxylapatit beschichtet ist.

8. Gewebekonnektor nach einem der Ansprüche 1 bis 3, bei dem der Träger (2) aus körperverträglichem Metall besteht.

9. Gewebekonnektor nach einem der Ansprüche 1 bis 8, bei dem in die Umfangskante des Trägers (2) radiale Schlitze (4) eingelassen sind.
